# EUROPEAN PATENT APPLICATION

(11) **EP 4 434 908 A1**
(43) Date of publication of application: **25.09.2024**
(21) Application number: 22891230.9
(22) Date of filing: 11.11.2022
(51) Int. Cl.: B65D 5/74, B65D 51/22, A61J 1/05, A61J 1/14, B65D 47/36, B65D 47/38, B65D 47/32, B65D 41/50, B65D 41/20, A61M 39/00, B65D 43/00

(54) **FIXED CLOSURE FOR LONG-LIFE PACKAGE WITH AN ACCESS FOR ENTERAL NUTRITION DEVICE FOR USE BY AN OPEN OR CLOSED SYSTEM**

(30) Priority: 15.11.2021 BR 102021022924
(71) Applicant: Santos Leite, Ronaldo, 88190-000 Governador Celso Ramos/SC (BR)
(72) Inventor: Santos Leite, Ronaldo, 88190-000 Governador Celso Ramos/SC (BR)
(74) Representative: Cyrson, Matthew Dominic
(86) International application number: PCT/BR2022/050435
(87) International publication number: WO 2023/081992

(57) **Abstract**

The present invention falls within the technical field of Food Packaging and has been specifically created to meet the market needs of Enteral Nutrition Foods, offering a single packaging solution that can be used for the two existing systems (Closed System and Open System). It is defined as an integrated opening device that is part of the aseptic carton packaging, which, in addition to the current functionality, allows its use by connecting to the enteral feeding set. Thus, the invention transforms the traditional aseptic carton packaging (used in open systems) into a multi-use packaging that can be utilized both in the traditional manner and as a closed system through the use of an enteral feeding set. This is because its features maintain the current functionality of the aseptic carton packaging, which breaks the internal protective seal upon opening, and also allow the connection with an enteral feeding set, preserving the seal, thus enabling its use in a closed system with protective characteristics identical to those of specialized closed system packaging available on the market.

## Description

### Field of the Invention

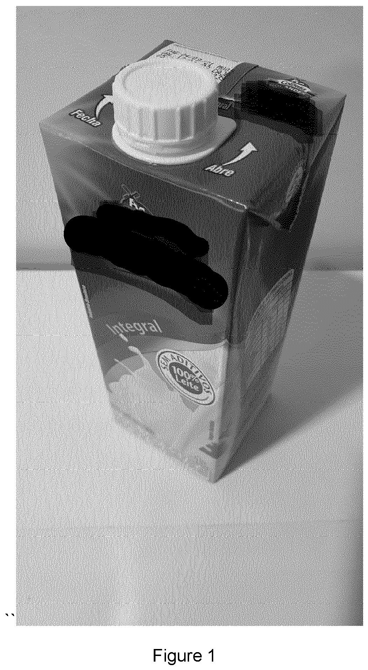

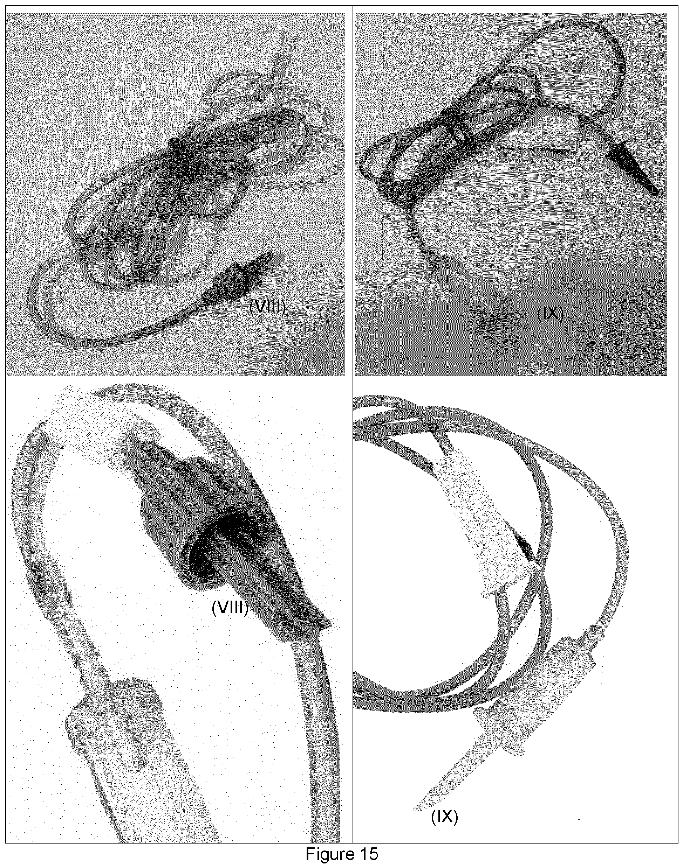

This patent application belongs to the technical field of food packaging. It is defined as a permanent attachment opening device for aseptic carton packaging, which, by replacing the current opening devices used in the market, transforms the traditional aseptic carton packaging (single use by open system) (Figure 1) into a multi-use packaging, which, optionally, can be used in two ways: by Open System, as it preserves the mechanisms present in the currently available models, or; as a Closed System, administered through the connection with an Enteral Feeding Set for use by tube, since the invention incorporates devices [(III) and (IV)] that allow the attachment of "Enteral Feeding Sets" (Figure 15).

### Background of the Invention

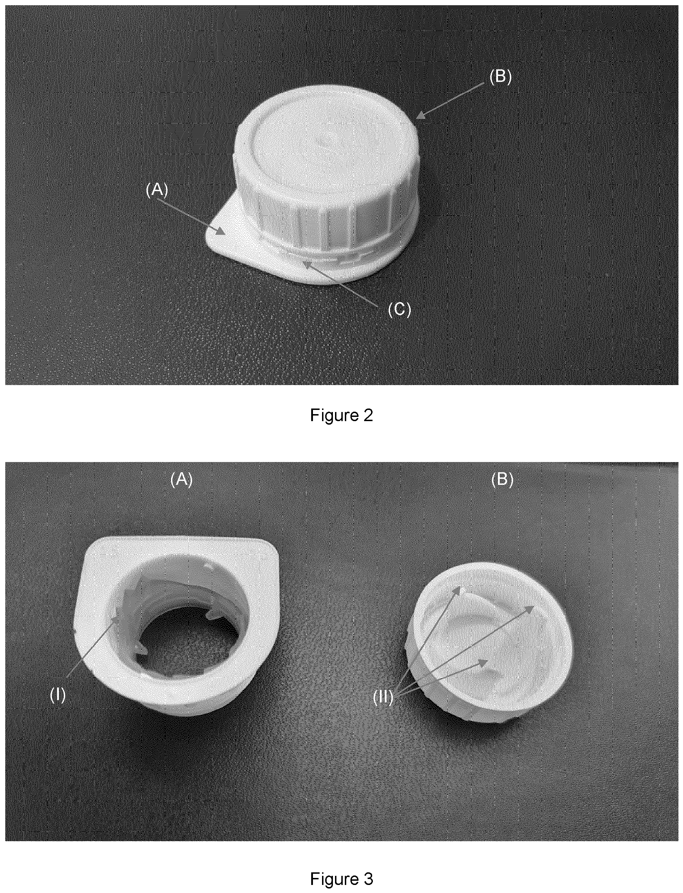

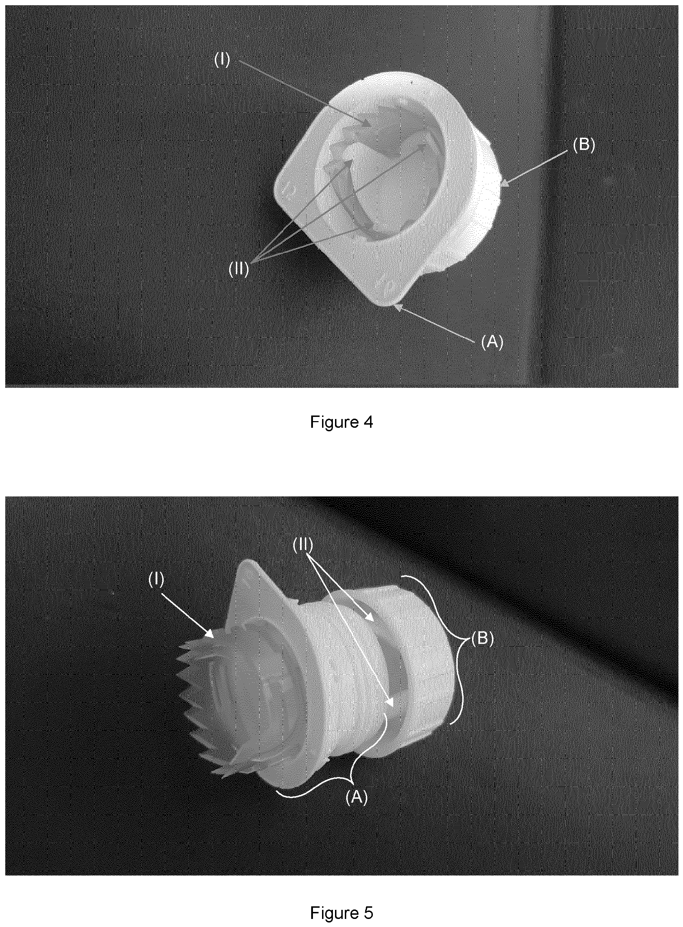

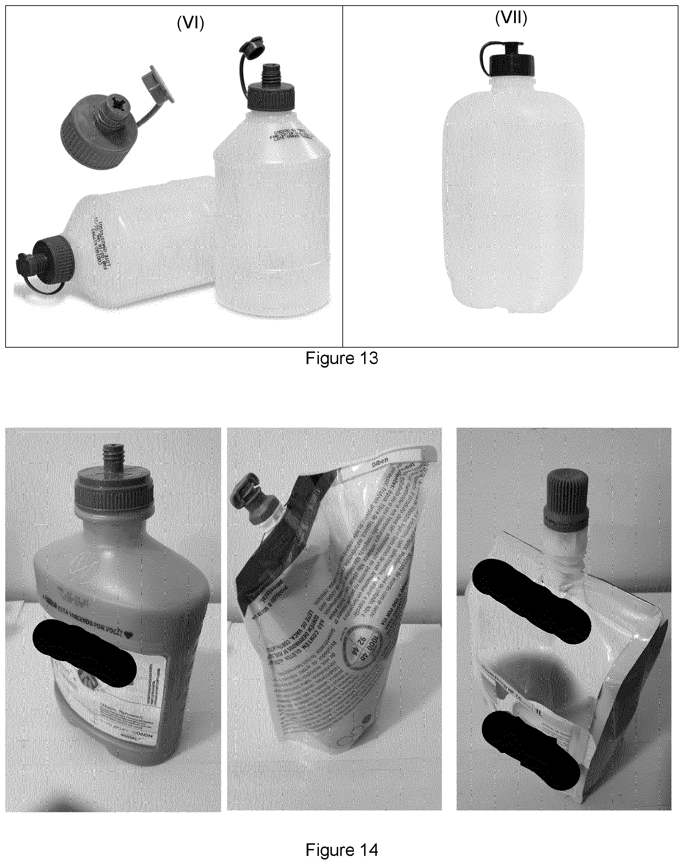

Enteral Nutrition Therapy can be performed using two types of systems: open and closed. In the open system, diets require handling and must be prepared in a specific location. The closed system, on the other hand, does not require preparation in specific areas. Enteral Nutrition Foods are currently available in both systems. The products for use in the open system, significantly cheaper, are offered in aseptic carton packaging. The products available in the closed system are offered in various types of packaging specially developed for this purpose (Figure 14). This invention allows the use of the aseptic carton packaging (Figure 1) as closed system packaging, without losing the main feature that facilitates its use as an open system, as it maintains the automatic seal opening through a cutting component (I) shown in Figures 3, 4, and 5. Introducing a new concept in packaging for enteral foods, as it presents in a single product the possibility of using both systems (open and closed). This is extremely advantageous in terms of cost compared to current closed system products, in addition to the practicality of delivering in a single product two solutions, adding greater security by eliminating the need for handling, and superior quality when compared to current products packaged in a closed system, which are sterilized by autoclaving.

Enteral Nutrition Therapy (ENT) has a high treatment cost for individuals, hospitals, health plans, or individuals treated in their own homes (Home Care). In cases where administration by Closed System is recommended, this expense can be tripled compared to the open system. This feature highlights that the packaging specially developed for the closed system (Figure 13) significantly impacts the final price of the product, making its use difficult or unfeasible for financially disadvantaged patients.

Currently, the foods packaged in aseptic carton packaging, which include open system enteral formulas, have opening devices whose use necessarily involves breaking the protective seal to allow the product's use, therefore excluding its use for the closed system, as this mandatory breach exposes the content, possibly leading to contamination by various agents, including manual contact or atmospheric air itself.

The present invention provide the current mechanisms [(I) and (II)] for cutting the protective seal; however, it enables its use through an Enteral Feeding Set with minimal violation of the protective seal, which can be minimally perforated by the Enteral Feeding Set (Figure 14) necessary for the administration of Enteral Nutrition via tube, which comes into contact with the product, keeping the content sufficiently preserved, in a manner equivalent to that in existing special packaging for enteral closed systems.

Thus, with the use of aseptic carton packaging adapted by this invention, it is possible to drastically reduce the cost of Enteral Foods for use by the Closed System and consequently the final consumer price, preserving the possibility of being used as an open system.

Besides the reduced price, it also stands out as a technical advantage that the nutritional characteristics of the packaged product are maintained, since the sterilization systems predominantly used in special closed system packaging (autoclaving process) are considerably more thermally aggressive than the process used for aseptic carton packaging (UHT process), preserving the more natural state of nutrients and reducing unwanted interactions between molecules promoted by the sterilization process. In other words, the invention not only enhances the mode of use but also reduces the cost and ensures a quality in the closed system that is identical to that found in the open system of aseptic carton packaging.

### Description of the Drawings

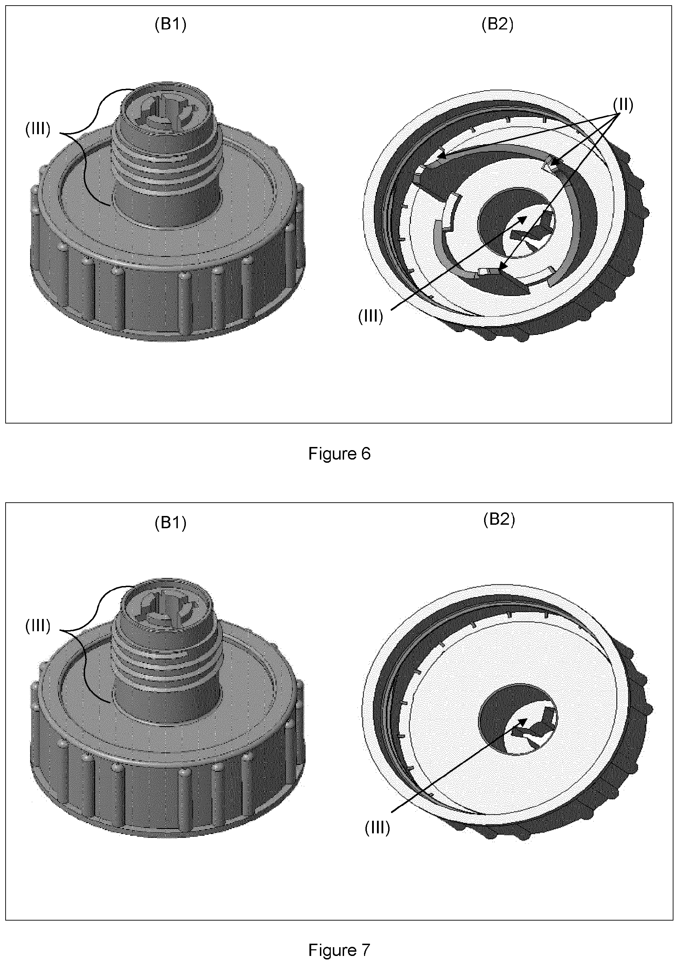

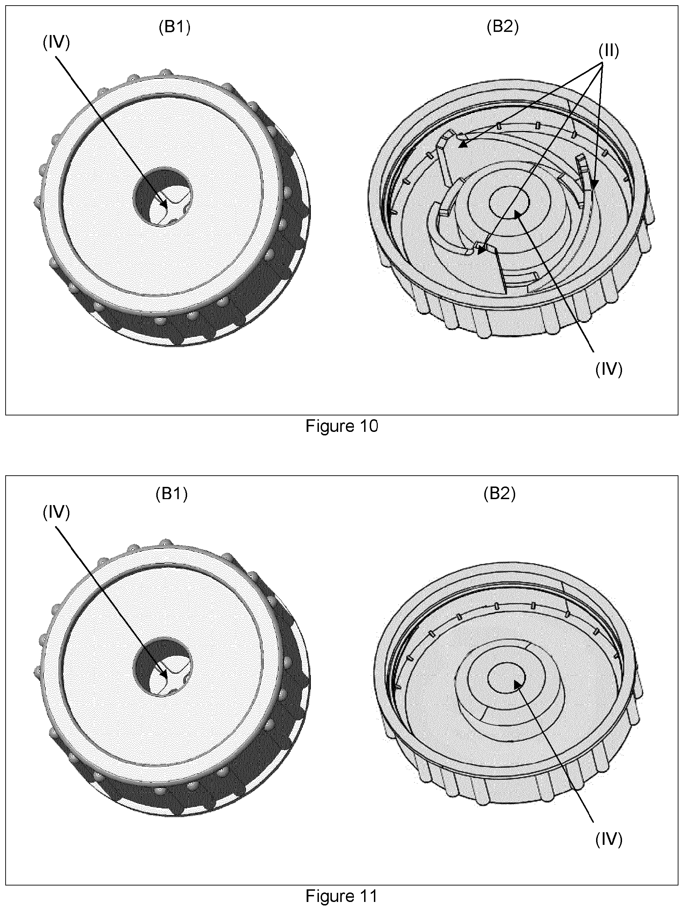

The invention can be materialized in various layouts with alternative mechanisms to accommodate various types of Enteral Feeding Sets (Figure 13) and complementary elements implemented to facilitate use and handling, ease production, and/or its application in the final packaging during the manufacturing of the product (Enteral Food). To better understand the concept of the invention, as well as the differences and similarities between the solutions available on the market, Figures 1 to 5 illustrate the opening device currently used in the market, displaying its mechanisms. Figures 6 to 12 illustrate the invention. Below is the description of the figures provided:
Figure 1 shows a photograph of the traditional aseptic carton packaging as currently used in the market.
Figure 2 displays the photograph of the opening device model currently used in traditional aseptic carton packaging.
Figure 3 presents the photograph of the lower (A) and upper (B) parts of the opening device currently used in aseptic carton packaging, arranged side by side.
Figure 4 shows the photograph of the opening device model currently used in aseptic carton packaging before being twisted open, i.e., with the intact seal (C).
Figure 5 displays the photograph of the opening device model currently used in aseptic carton packaging after the cap is unscrewed, with the seal cutting component (I) projected out.
Figure 6 presents the design of the prototype for the upper part (B) of the opening device with access for a cross-type Enteral Feeding Set (III), for packaging with automatic seal cutting (contains structure II), top view (B1) and bottom view (B2).
Figure 7 shows the design of the prototype for the upper part (B) of the opening device with access for a cross-shaped Enteral Feeding Set (III), for packaging with manual seal removal. Top view (B1) and bottom view (B2).
Figure 8 displays the design of the prototype for the upper part (B) of the opening device with access for a spike-shaped Enteral Feeding Set (IV), designed from bottom to top, for packaging with automatic seal cutting (contains structure II), top view (B1) and bottom view (B2).
Figure 9 presents the design of the prototype for the upper part of the opening device (B) with access for a cross-shaped Enteral Feeding Set (IV), designed from bottom to top, for packaging with manual seal removal. Top view (B1) and bottom view (B2).
Figure 10 shows the design of the upper part (B) of the opening device with access (IV)for a spike-shaped Enteral Feeding Set, designed from top to bottom, for packaging with automatic seal cutting (contains structure II), top view (B1) and bottom view (B2).
Figure 11 presents the design of the upper part (B) of the opening device with access (IV) for a cross-shaped Enteral Feeding Set, designed from top to bottom, for packaging with manual seal removal. Top view (B1) and bottom view (B2).
Figure 12 displays the design of the upper parts (B) of the opening devices with microbiological filters (V) for internal decompression (top view B1).
Figure 13 shows photographs exemplifying two models of bottles used to administer products from the open system with cross-type (VI) and lancet-type (VII) accesses.
Figure 14 presents photographs exemplifying some models of special packaging used in closed system products currently available in the market.
Figure 15 displays a photograph exemplifying two Enteral Feeding Sets with cross-shaped (VIII) and spike-shaped (IX) tips.

The components present in layouts of the invention are described below:
(A) - Base (lower part) of the opening device
(B) - Upper part of the opening device. (B1) Top view, (B2) Bottom view
(C) - Tamper-evident seal of the opening device
   (I) - Mechanism for cutting the internal seal of the aseptic carton packaging
   (II) - Auxiliary structure that rotates the cutting mechanism (I)
   (III) - Device for fixing the cross-shaped Enteral Feeding Set
   (IV) - Device for fixing the spike-shaped Enteral Feeding Set
   (V) - Microbiological filter for internal decompression

### Description of the Invention

The opening device consists of two parts, the base/lower part (A) and the cap/upper part (B). It should be noted that the invention incorporates mechanisms at the base (A) equivalent to those found in the existing opening devices (Figures 2, 3, 4, and 5). That is, the base (A) maintains the structure of the opening devices currently available on the market and includes the "automatic cutting mechanism" (I) in models with automatic seal cutting (Figures 6, 8, and 10). Thus, all the differences that characterize the concept of this invention occur mainly in the upper part (B) of the opening device, as demonstrated in figures 6 to 12. In Figures 6, 8, and 10, it is possible to observe that the upper part (B) contains an "auxiliary structure" (II) that rotates the "automatic cutting mechanism" (I) present in the base, giving this opening device the optional functionality of automatic cutting for use as an open system, simply by unscrewing the cap to open.

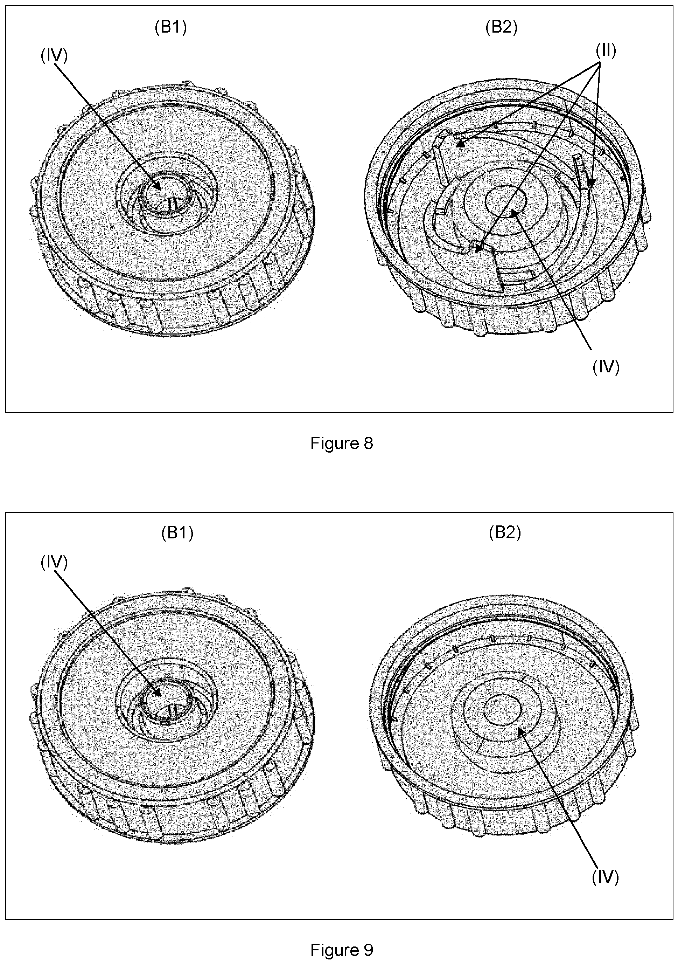

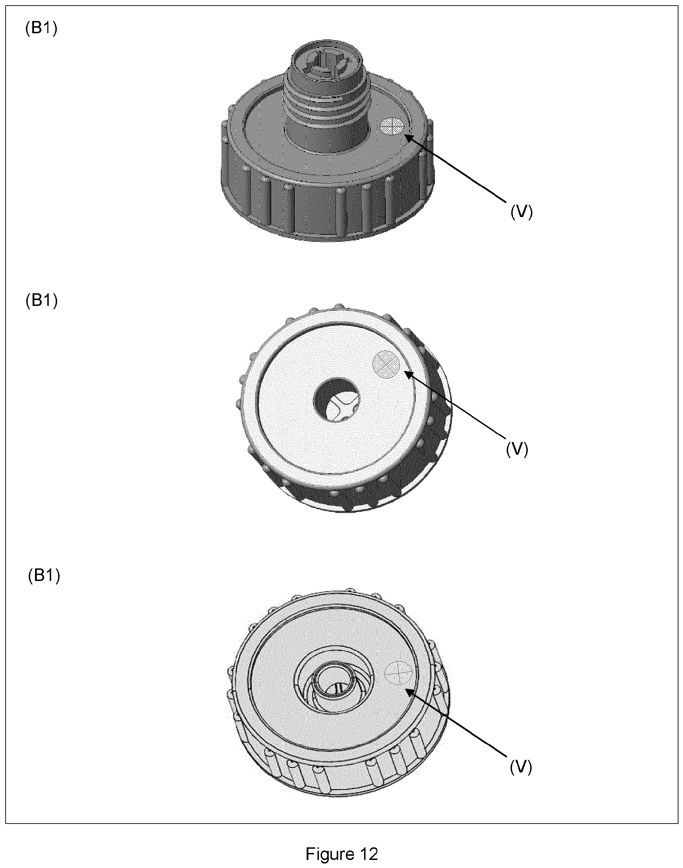

This model of the opening device will be an integral part of the aseptic carton packaging and, as demonstrated in the figures explained above, can be materialized in various proposals, whether to fit the different types of Enteral Feeding Sets present in the market, to facilitate the production of the model, or to improve the handling experience through more practical, comfortable, and safe models in terms of use and preservation of product quality during administration. To illustrate compatibility with different models of Enteral Feeding Sets, the prototypes were presented for two types: Cross-shaped Enteral Feeding Set (Figures 6 and 7); Spike-shaped Enteral Feeding Set (Figures 8, 9, 10, and 11). For the various types of Enteral Feeding Sets, the possibilities of implementation illustrated through different layouts can be applied with or without a biological filter for air intake as shown in the images in Figure 12.

This invention, an opening device of an aseptic carton packaging, regardless of the layout, can be used in two ways, for the administration of enteral nutrition via an open system or a closed system. Thus, the functionality of the open system of the current aseptic carton packaging will be provide, with the presence of components for automatic seal cutting [(I) and (II)], and devices will be added that allow its use as a closed system, common in special packaging: Entry for Enteral Feeding Set (Figure 14), whether Cross-shaped (III), Spike-shaped (IV), or other compatible types; Microbiological filter (V) to facilitate the flow of product through the tube (Figure 12), allowing the entry of filtered air to balance the internal pressure of the packaging. The microbiological filter can be eliminated in packaging that collapses under pressure, maintaining satisfactory flow during the administration of the product. For this reason, we present designs that demonstrate the layout of prototypes with a filter (Figure 12).

The inventive concept can be illustrated by the design of the prototype (Figure 6) used with the base (A) represented in Figure 3, being an "opening device for aseptic carton packaging with access for a cross-shaped Enteral Feeding Set on its upper part." This device consists of a base [Figure 3 (A)] and an upper part (Figure 6). The upper part contains an auxiliary structure that rotates the cutting mechanism (I) and is innovated with the presence of the device (III) for fixing a cross-type Enteral Feeding Set. The base is connected to the upper part by a tamper-evident seal (C), which, for use as an open system, is broken by twisting the cap to open the packaging, breaking the internal seal and allowing the contents to be poured into another container for oral use.

### Examples of Embodiments of the Invention

Besides the proposal described above, some examples of implementations of the invention are represented in Figures 7, 8, 9, 10, and 11. All embodiments can also be produced with a filter (Figure 12). However, due to the ease of manufacturing and functionality, the form described in the previous paragraph can be initially produced.

Most foods packaged in aseptic carton packaging feature an opening device; however, all models of opening devices used so far in such packaging lack means for attaching an Enteral Feeding Set. Thus, this invention represents an unprecedented solution for the enteral nutrition market, as it enables conventional aseptic carton packaging, widely used in the food market, to broaden its application to the enteral nutrition market, maintaining its use as an open system and expanding the possibilities for use in closed enteral systems.

## Claims

1. OPENING DEVICE FOR ASEPTIC CARTON PACKAGING USED FOR ENTERAL NUTRITION THROUGH CLOSED SYSTEM OR OPEN SYSTEM, **characterized by** adding the possibility of using traditional long-life packaging as packaging for use via feeding tube in a Closed System with the use of a cross-shaped Enteral Feeding Set (Figure 6) for packaging models with automatic protective seal cutting, having its fixed base [Figure 3 (A)] accompanied by a mechanism for cutting the internal seal (I), and its upper part (B) containing an auxiliary structure to rotate the cutting mechanism (I) and a device for fixing a cross-shaped Enteral Feeding Set (III), without a microbiological filter.

2. OPENING DEVICE FOR ASEPTIC CARTON PACKAGING USED FOR ENTERAL NUTRITION THROUGH CLOSED SYSTEM OR OPEN SYSTEM, according to Claim 1, **characterized by** adding the possibility of using traditional long-life packaging as packaging for use via feeding tube in a Closed System with the use of a cross-shaped Enteral Feeding Set (Figure 7) for packaging models with manual removal of protective seal, having a fixed base [Figure 3 (A)], and its upper part (B) containing a device for fixing a cross-shaped Enteral Feeding Set (III), without a microbiological filter..

3. OPENING DEVICE FOR ASEPTIC CARTON PACKAGING USED FOR ENTERAL NUTRITION THROUGH CLOSED SYSTEM OR OPEN SYSTEM, according to Claim 1, **characterized by** adding the possibility of using traditional long-life packaging as packaging for use via feeding tube in a Closed System with the use of a cross-shaped Enteral Feeding Set (Figure 6) for packaging models with automatic protective seal cutting, having its fixed base [Figure 3 (A)] accompanied by a mechanism for cutting the internal seal (I), and its upper part (B) containing an auxiliary structure to rotate the cutting mechanism (I) and a device for fixing a cross-shaped Enteral Feeding Set (III), with a microbiological filter (IV) as shown in Figure 12.

4. OPENING DEVICE FOR ASEPTIC CARTON PACKAGING USED FOR ENTERAL NUTRITION THROUGH CLOSED SYSTEM OR OPEN SYSTEM, according to Claim 2, **characterized by** adding the possibility of using traditional long-life packaging as packaging for use via feeding tube in a Closed System with the use of a cross-shaped Enteral Feeding Set (Figure 7) for packaging models with manual removal of protective seal, having a fixed base [Figure 3 (A)], and its upper part (B) containing a device for fixing a cross-shaped Enteral Feeding Set (III), with a microbiological filter (IV) as shown in Figure 12.

5. OPENING DEVICE FOR ASEPTIC CARTON PACKAGING USED FOR ENTERAL NUTRITION THROUGH CLOSED SYSTEM OR OPEN SYSTEM, according to Claim 1, **characterized by** adding the possibility of using traditional long-life packaging as packaging for use via feeding tube in a Closed System with the use of a spike-shaped Enteral Feeding Set (Figure 8) for packaging models with automatic protective seal cutting, having its fixed base [Figure 3 (A)] accompanied by a mechanism for cutting the internal seal (I), and its upper part (B) containing an auxiliary structure to rotate the cutting mechanism (I) and a device for fixing a spike-shaped Enteral Feeding Set (IV) designed from bottom to top, without a microbiological filter.

6. OPENING DEVICE FOR ASEPTIC CARTON PACKAGING USED FOR ENTERAL NUTRITION THROUGH CLOSED SYSTEM OR OPEN SYSTEM, according to Claim 2, **characterized by** adding the possibility of using traditional long-life packaging as packaging for use via feeding tube in a Closed System with the use of a spike-shaped Enteral Feeding Set (Figure 9) for packaging models with manual removal of protective seal, having a fixed base [Figure 3 (A)], and its upper part (B) containing a device for fixing a spike-shaped Enteral Feeding Set (IV) designed from bottom to top, without a microbiological filter.

7. OPENING DEVICE FOR ASEPTIC CARTON PACKAGING USED FOR ENTERAL NUTRITION THROUGH CLOSED SYSTEM OR OPEN SYSTEM, according to Claim 5, **characterized by** adding the possibility of using traditional long-life packaging as packaging for use via feeding tube in a Closed System with the use of a spike-shaped Enteral Feeding Set (Figure 8) for packaging models with automatic protective seal cutting, having its fixed base [Figure 3 (A)] accompanied by a mechanism for cutting the internal seal (I), and its upper part (B) containing an auxiliary structure to rotate the cutting mechanism (I) and a device for fixing a spike-shaped Enteral Feeding Set (IV) designed from bottom to top, with a microbiological filter (IV) as shown in Figure 12.

8. OPENING DEVICE FOR ASEPTIC CARTON PACKAGING USED FOR ENTERAL NUTRITION THROUGH CLOSED SYSTEM OR OPEN SYSTEM, according to Claim 6, **characterized by** adding the possibility of using traditional long-life packaging as packaging for use via feeding tube in a Closed System with the use of a spike-shaped Enteral Feeding Set (Figure 9) for packaging models with manual removal of protective seal, having a fixed base [Figure 3 (A)], and its upper part (B) containing a device for fixing a spike-shaped Enteral Feeding Set (IV) designed from bottom to top, with a microbiological filter (IV) as shown in Figure 12.

9. OPENING DEVICE FOR ASEPTIC CARTON PACKAGING USED FOR ENTERAL NUTRITION THROUGH CLOSED SYSTEM OR OPEN SYSTEM, according to Claim 5, **characterized by** adding the possibility of using traditional long-life packaging as packaging for use via feeding tube in a Closed System with the use of a spike-shaped Enteral Feeding Set (Figure 10) for packaging models with automatic protective seal cutting, having its fixed base [Figure 3 (A)] accompanied by a mechanism for cutting the internal seal (I), and its upper part (B) containing an auxiliary structure to rotate the cutting mechanism (I) and a device for fixing a spike-shaped Enteral Feeding Set (IV) designed from top to bottom, without a microbiological filter.

10. OPENING DEVICE FOR ASEPTIC CARTON PACKAGING USED FOR ENTERAL NUTRITION THROUGH CLOSED SYSTEM OR OPEN SYSTEM, according to Claim 6, **characterized by** adding the possibility of using traditional long-life packaging as packaging for use via feeding tube in a Closed System with the use of a spike-shaped Enteral Feeding Set (Figure 11) for packaging models with manual removal of protective seal, having a fixed base [Figure 3 (A)], and its upper part (B) containing a device for fixing a spike-shaped Enteral Feeding Set (IV) designed from top to bottom, without a microbiological filter.

11. OPENING DEVICE FOR ASEPTIC CARTON PACKAGING USED FOR ENTERAL NUTRITION THROUGH CLOSED SYSTEM OR OPEN SYSTEM, according to Claim 9, **characterized by** adding the possibility of using traditional long-life packaging as packaging for use via feeding tube in a Closed System with the use of a spike-shaped Enteral Feeding Set (Figure 10) for packaging models with automatic protective seal cutting, having its fixed base [Figure 3 (A)] accompanied by a mechanism for cutting the internal seal (I), and its upper part (B) containing an auxiliary structure to rotate the cutting mechanism (I) and a device for fixing a spike-shaped Enteral Feeding Set (IV) designed from top to bottom, with a microbiological filter (IV) as shown in Figure 12.

12. OPENING DEVICE FOR ASEPTIC CARTON PACKAGING USED FOR ENTERAL NUTRITION THROUGH CLOSED SYSTEM OR OPEN SYSTEM, according to Claim 10, **characterized by** adding the possibility of using traditional long-life packaging as packaging for use via feeding tube in a Closed System with the use of a spike-shaped Enteral Feeding Set (Figure 11) for packaging models with manual removal of protective seal, having a fixed base [Figure 3 (A)], and its upper part (B) containing a device for fixing a spike-shaped Enteral Feeding Set (IV) designed from top to bottom, with a microbiological filter (IV) as shown in Figure 12.
